(19) Europäisches Patentamt
European Patent Office
Office européen des brevets

(11) Publication number: **0 170 836**
**B1**

(12) **EUROPEAN PATENT SPECIFICATION**

(45) Date of publication of patent specification: **01.03.89**

(51) Int. Cl.[4]: **C 07 D 301/19**, C 07 D 303/04, B 01 J 21/02

(21) Application number: **85107431.0**

(22) Date of filing: **14.06.85**

# (54) Method for preparation of styrene oxides.

(30) Priority: **14.06.84 JP 122671/84**

(43) Date of publication of application: **12.02.86 Bulletin 86/07**

(45) Publication of the grant of the patent: **01.03.89 Bulletin 89/09**

(84) Designated Contracting States: **DE FR GB**

(58) References cited:
**DE-B-1 468 012**
**DE-B-1 593 305**
**DE-B-1 593 306**
**US-A-3 832 363**
**US-A-3 843 694**

**CHEMICAL ABSTRACTS, vol. 83, no. 19, 10th November 1975, page 532, abstract no. 163981y, Columbus, Ohio, US; J.S. McINTYRE et al.: "Epoxidation of 2,4,4-trimethyl-1-pentene", & CA - A - 967 973 (DOW CHEMICAL CO.) 20-05-1975**

(73) Proprietor: **MITSUBISHI PETROCHEMICAL CO., LTD.**
**5-2, 2-chome, Marunouchi**
**Chiyoda-ku Tokyo 100 (JP)**

(72) Inventor: **Imanari, Makoto**
**1315, Ohazawakaguri Ami-machi**
**Inashiki-gun Ibaraki (JP)**
Inventor: **Iwane, Hiroshi**
**1315, Ohazawakaguri Ami-machi**
**Inashiki-gun Ibaraki (JP)**
Inventor: **Ohtaka, Satoshi**
**1315, Ohazawakaguri Ami-machi**
**Inashiki-gun Ibaraki (JP)**
Inventor: **Aoki, Tadmichi**
**1315, Ohazawakaguri Ami-machi**
**Inashiki-gun Ibaraki (JP)**

(74) Representative: **Lehn, Werner, Dipl.-Ing. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 170 836 B1

(56) References cited:
CHEMICAL ABSTRACTS, vol. 90, no. 19, 7th May 1979, page 592, abstract no. 151959d, Columbus, Ohio, US; M. HABA et al.: "Epoxidation of conjugated olefins", & JP - A - 53 92704 (NIPPON OILS AND FATS CO., LTD.) 15-08-1978

CHEMICAL ABSTRACTS, vol. 90, no. 23, 4th June 1979, page 588, abstract no. 186058m, Columbus, Ohio, US; A. BADEV et al.: "Catalytic effect of boron additives in oxidation of unsaturated compounds", & KHIM. IND. (Sofia) 1978, (10), 435-440

CHEMICAL ABSTRACTS, vol. 84, no. 9, 1st March 1976, page 484, abstract no. 59066p, Columbus, Ohio, US; A.T. BADEV et al.: "Production of epoxystyrene by direct liquid-phase oxidation in the presence of boron promoters", & REV. CHEM. (Bucharest) 1975, 26(4), 278-282

**Description**

The present invention relates to a method for preparation of styrene oxides, more precisely, to a method for preparation of styrene oxides by reacting a styrene and an organic peroxide in the presence of an epoxidation catalyst and a boron compound.

Styrene oxides are useful as raw materials for stabilizers of high molecular compounds, ultraviolet ray absorbents, medicines, perfumes stabiolizers of solvents and food additives. In particular, they are important as raw materials of perfumes, and as raw materials for phenylalanines or other amino acids.

Some methods have heretofore been known for preparation of styrene oxides by reacting a styrene and an organic peroxide in the presence of an epoxidation catalyst.

However, conventional known methods are defective in that radical polymerization of the used styrene compound occurs due to the radicals formed by partial pyrolysis of the used organic peroxide during the reaction, resulting in decrease of the selectivity of the formed styrene oxide compound to the used styrene compound, and that the conversion of the used organic peroxide is not sufficiently high, resulting in complication of post-treatment and purification steps.

Some other methods have been proposed in order to overcome said defects, for example, as follows: The reaction is carried out in the presence of an aliphatic or alicyclic monoamine and an epoxidation catalyst (Japanese Patent Publication 8106/82), or otherwise, the reaction is carried out in the presence of an organic amine compound and an epoxidation catalyst (Japanese Patent Application 133279/81.

These methods have a common technique to use an amine compound. However, these are not still industrially advantageous in that the selectivity of the formed styrene oxide compounds to the used styrene compounds as well as the conversion of the used organic peroxides is not sufficiently high.

In Chemical Abstracts, Volume 90, No. 19, (1979) page 592, Abstract No. 151959d, epoxidation of 2,4,4-trimthyl-1-pentene is described. Certain co-catalysts like $H_3BO_3$, $B_2O_3$, $B(OEt)_3$ or tri-o-cresyl borate are used together with cumene hydroperoxide and Mo naphthenane to increase the selectivity and speed of epoxidation of butadiene. A similar reaction is described in Chemical Abstracts, Volume 83, No. 19 (1975), page 532, Abstract No. 16398ly. Furthermore, the catalytic epoxidation of olefins is described in US—A—3 843 694. This epoxidation is carried out by reacting an organic peroxide, an epoxidation catalyst and $BPO_4$ with an ethylenically unsaturated compound. Although styrene is mentioned as an ethylenically unsaturated compound, the reaction is described only for the preparation of 2,3-epoxy-2-methylbutane.

The present inventors have repeated various studies in an effort to obtain an industrially advantageous method for preparing the aimed styrene oxide compounds with high yield, and have at last attained the present invention.

The method of the present invention is characterized by excellent features that:

(1) the selectivity of styrene oxide formation in relation to styrene consumption and to organic peroxides consumption is high;

(2) the conversion of organic peroxides is high, and the epoxidation speed is high.

The present invention comprises a method for preparation of styrene oxides by reacting a styrene and an organic peroxide in the presence of an epoxidation catalyst and a boron compound, which is characterized in that said boron compound is selected from borates of formula (I) or metaborates of formula (II) or a mixture thereof

$$B(OR^1)_3 \qquad (I)$$

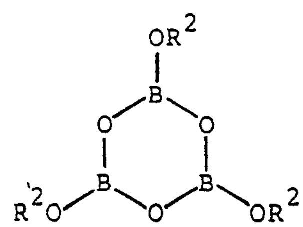

(II)

wherein $R^1$ and $R^2$ are an alkyl group having 1 to 10 carbon atoms, an aralkyl group having 7 to 20 carbon atoms or an aryl group having 6 to 20 carbon atoms.

*Epoxidation Catalysts:*

Epoxidation catalysts to be used in the present invention are metal compounds selected from molybdenum compounds, vanadium compounds and titanium compounds, which are known as an epoxidation catalyst for olefins, for example, including oxides, halides, naphthenates, stearates, octanoates, carbonyl complexes and acetylacetonates of said metals.

Examples of said metal compounds are molybdenum compounds including inorganic molybdenum compounds such as sodium molybdate, molbdenum oxide, molybdenum sulfide, phosphomolybdic acid,

sodium phosphomolybdate, molybdenum hexacarbonyl, molybdenum pentachloride, ammonium molybdate, ammonium paramolybdate and silicomolybdic acid, and molybdenum-organic acid salts such as molybdenum oxide acetylacetonate, monybdenum acetylacetonate, molybdenum naphthenate, molybdenum stearate and molybdenum octanoate; vanadium compounds such as sodium vanadate, vanadium oxide, vanadium oxy-trichloride and vanadium oxide acetylacetonate; and titanium compounds such as titanium trichloride and titanium oxide.

Molybdenum compounds are preferred among them, molydenum compounds which are soluble in an organic solvent are more preferred, and molybdenum oxide acetylacetonate, molybdenum hexacarbonyl, molybdenum naphthenate and molybdenum octanoate are particularly preferred.

The amount of said epoxidation catalyst to be used is 0.00001 to 0.1 mol, preferably 0.0001 to 0.01 mol, to 1 mol of the organic peroxide to be reacted.

*Boron Compounds:*

Borates and metaborates are used as boron compounds. The borates are represented by $B(OR^1)_3$, wherein $R^1$ is an alkyl group having 1 to 10 carbon atoms, an aralkyl group having 7 to 20 carbon atoms or an aryl group having 6 to 20 carbon atoms.

Examples of said borates are trimethyl borate, triethyl borate, tripropyl borate, tributyl borate, triphenyl borate, tris{2-[2-(2-naphthyl])propyl]} borate, tris{2-[2-(1-naphthyl)propyl]} borate, tris{1-[1-(2-naphtyl)ethyl]} borate, tris{1-[1-(1-naphthyl)ethyl]} borate, tris{2-[2-(4-biphenylyl)propyl]} borate, tris{1-[1-(4-biphenylyl)ethyl]} borate, tris{2-[2-(2-biphenyl)propyl]} borate and tris{1-[1-(2-biphenyl)ethyl]} borate.

The metaborates are represented by the following formula:

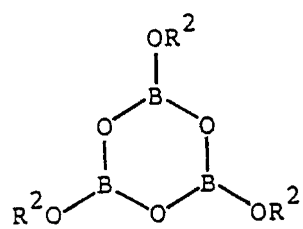

(II)

wherein $R^2$ is an alkyl group having 1 to 10 carbon atoms, an aralkyl group having 7 to 20 carbon atoms or an aryl group having 6 to 20 carbon atoms.

Examples of said metaborates are methyl metaborate, ethyl metaborate, propyl metaborate, butyl metaborate, cyclohexyl metaborate, phenyl metaborate, {2[2-(2-naphthyl)propyl]} metaborate, {1-[1-(1-naphthyl])ethyl]} metaborate, {2-[2-(4-biphenylyl)propyl]} metaborate and {2-[2-(4-biphenylyl)ethyl]} metaborate.

These boron compounds may be used singly or in the form of a mixture of two or more compounds.

Among the above described boron compounds, borates and metaborates of said formulae wherein $R^1$ and $R^2$ are an alkyl group having 3 to 10 carbon atoms or an aralkyl group having 8 to 20 carbon atoms are preferred, especially $R^1$ or $R^2$ which is an α-secondary or α-tertiary alkyl group having 3 to 10 carbon atoms or an α-secondary or α-tertiary aralkyl group having 8 to 20 carbon atoms is preferred, and those borates are particularly preferred, for example, including triisopropyl borate, tri-tert-butyl borate, tri-sec-butyl borate and tricumyl borate.

The amount of said boron compound to be used is not specifically limitative, and is generally 10 to 100 mols, preferably 30 to 80 mols, to 1 mol of said epoxidation catalyst.

*Styrenes:*

Styrenes which may be used in the present invention are represented by the following formula:

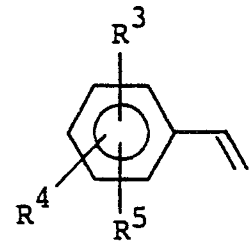

wherein $R^3$, $R^4$ and $R^5$ may be the same or different and independently represent hydrogen, an alkyl group having 1 to 10 carbon atoms, an aryl group having 6 to 15 carbon atoms, an alkoxy group having 1 to 10

carbon atoms, a phenoxy group having 6 to 15 carbon atoms, a hydroxyl group, a halogen atom, a nitro group, an acyl group or an amono group.

These styrenes are easily available, for example, by:

(1) dehydrogenation of corresponding ethylbenzenes, or

(2) reduction and dehydration of corresponding acetophenones.

The purity of said styrenes is not limitative so far as they do not badly affect the reaction, and is preferably 80% or more. Examples of said styrenes are styrene, methoxystyrene, methylstyrene, dimethylstyrene, ethylstyrene, isopropylstyrene, isobutylstyrene, vinylphenol, 3-chloro-4-allyloxystyrene, chlorostyrene, bromostyrene, vinyl-biphenyl, 3,4,5-trimethoxystyrene, phenoxystyrene and dimethylaminostyrene.

*Organic Peroxides:*

Organic peroxides which may be used in the present invention are those represented by $R^6OOH$, wherein $R^6$ is an alkyl or aralkyl group having 1 to 15 carbon atoms.

Examples of said organic peroxides are ethylbenzene-hydroperoxide, cumene-hydroperoxide, methanehydroperoxide, diisopropylbenzene-monohydroperoxide, diisopropylbenzene-dihydroperoxide, tetralinhydroperoxide, cyclopentyl-hydroperoxide, cyclohexyl-hydroperoxide and t-butyl-hydroperoxide.

These organic peroxides may be used in the form of high concentration of high purity, or alternatively may be used, as diluted with a solvent. One preferred embodiment is a by-product oil containing about 10 to 90 wt% of cumene-hydroperoxide, which may be obtained as a by-product (or an intermediate) in a process for preparation of phenol and acetone by catalytic oxidation of cumene.

Commercial organic peroxides are in general stabilized, as containing an alkaline substance such as sodium hydroxide of sodium carbonate. However, it is preferred in the organic peroxide compounds to be used in the present invention that the content of such alkaline substance is as low as possible, and those containing no alkaline substance additive is most preferred.

In the process of the present invention, the amount of styrenes to be used is 1 to 30 mols, preferably 1 to 10 mols, to 1 mol of said organic peroxide.

*Other Additives:*

In the present invention, styrene oxides may be obtained by reaction of the above mentioned styrene and organic peroxide in the presence of the above mentioned epoxidation catalyst and boron compound. To this reaction system, the following compounds may further be added as additives, in addition to the above mentioned substances, so far as they do not badly affect the object of the present invention, for example, in an amount of 5 to 50 mols, preferably 10 to 40 mols, to 1 mol of the epoxidation catalyst.

Examples of such additives are organic amine compounds such as butylamine, octylamine, nonylamine, diethylamine, triethylamine, ethylenediamine, aniline, N,N-dimethylaniline, pyridine and picoline; phenol compounds such as hydroquinone, t-butylcatechol, and di-t-butylhydroxytoluene; organic nitroso compounds such as N-nitrosodiphenylamine, nitrosobenzene and N-nitrosodiethylaniline; and alkali metal salts such as sodium acetate, potassium acetate, lithium acetate, and sodium acetylacetonate.

*Reaction:*

The process of the present invention may be carried out in the absence of a solvent, but is preferably carried out in the presence of a solvent as the pyrolysis reaction of the used organic peroxide is an exothermic reaction and thus run-away reaction and styrene polymerization reaction must be prevented.

Solvents which may be used in the present invention are not specifically limitative and any and every inert solvent may be used. In particular, hydrocarbon solvents such as aromatic hydrocarbons including benzene, toluene, ethylbenzene and cumene; aliphatic hydrocarbons including hexane, heptane and octane; and alicyclic hydrocarbons including cyclohexane and cyclododecane are preferred. Said aromatic hydrocarbon solvents are especially preferred among them. The amount of the solvent is not specifically limitative and is in general such that the concentration of the used organic peroxide is to be 5 to 60 wt%, more preferably 5 to 40 wt%, to the reaction solution, in view of economical point and of easiness of isolation and purification.

The reaction temperature and the reaction period vary depending upon the kind of the used organic peroxide, and in general, the reaction temperature is preferably 70 to 170°C, and the reaction period is preferably 0.1 to 120 minutes; and the reaction temperature is more preferably 90 to 150°C and the reaction period is more preferably 0.5 to 30 minutes, in order to attain higher selectivity of the formed styrene oxide compound to the used styrene compound and higher conversion of the used organic peroxide compound.

The process of the present invention may be carried out by any of batchwise method and continuous method. In particular, when such conditions are selected that the reaction temperature is higher and the reaction period is shorter, the latter continuous method is preferred, as the reaction of the present process is an exothermic reaction.

In this case, the above mentioned reaction period indicates the time while the reaction solution remains in a reaction tube, passing therethrough. Accordingly, when a reaction solution feeding speed is A (ml/min) and the capacity of the reaction tube is B (ml), then the reaction time is (B/A) minutes.

Reaction products obtainable by the present process comprise a styrene oxide compound and a

hydroxyl-containing compound corresponding to the used organic peroxide. For example, when cumene-hydroperoxide is used, cumyl-alcohol is obtained together with a styrene oxide compound, and analogously, when ethylbenzene-hydroperoxide is used, 1-phenylethyl alcohol is obtained, and when t-butylhydroperoxide is used, t-butyl alcohol is obtained, every together with a styrene oxide compound.

Said cumyl-alcohol, 1-phenylethyl-alcohol and t-butyl alcohol are per se useful compounds, and after dehydrated, α-methylstyrene, styrene and isobutylene are obtained, respectively, which are also useful compounds.

These reaction products are isolated and purified by distillation.

The present invention will be explained in greater detail by reference to the following Examples. Unless otherwise indicated, all percents are by mol. Conversion and selectivity are defined according to the following formulae:

Conversion (%) of organic peroxide =

$$\frac{\text{Amount of reacted organic peroxide (mols)}}{\text{Amount of organic peroxide fed in the reaction system (mols)}} \times 100$$

Selectivity (%) to styrene compound =

$$\frac{\text{Amount of formed styrene oxide compound (mols)}}{\text{Amount of reacted styrene compound (mols)}} \times 100$$

Selectivity (%) to organic peroxide =

$$\frac{\text{Amount of formed styrene oxide compound (mols)}}{\text{Amount of reacted organic peroxide (mols)}} \times 100$$

## Examples 1—6

A cumene solution containing 20 wt% of cumene-hydroperoxide, which was a by-product produced in a process of preparing phenol and acetone by catalytic oxidation of cumene and which did not contain any stabilizer of an alkaline substance, in an amount of 2,900 g/hour; 1.9 times mol/hour of styrene on the basis of said cumene-hydroperoxide, in an amount of 775 g/hour; 10 wt% solution of molybdenum octanoate in an amount of 1/3,000 mol/hour; and a borate as listed in the following Table 1, in a molar ratio as determined therein, were fed into a reaction tube made of stainless steel having an inner diameter of 4 mm and a length of 60 m and reacted at a temperature of 115°C, while passing therethrough within a retention time of 10 minutes.

The conversion of the cumene-hydroperoxide in the reaction products was determined by iodometry; and the amount of each of the non-reacted styrene and reaction products was determined by quantitative analysis with gas chromatography. Results are given in Table 1.

TABLE 1

| Example No. | Borate | CHP/B (molar ratio) | Conversion of Cumene-Hydroperoxide (%) | Selectivity of Styrene Oxide To Styrene (%) | Selectivity of Styrene Oxide To Cumene-Hydroperoxide (%) |
|---|---|---|---|---|---|
| 1 | Triisopropyl Borate | 75 | 99.2 | 96.5 | 92.0 |
| 2 | " | 100 | 99.0 | 95.2 | 90.3 |
| 3 | Tri-tert-butyl Borate | 100 | 99.3 | 97.0 | 91.8 |
| 4 | Trimethyl Borate | 40 | 98.7 | 88.1 | 88.5 |
| 5 | " | 75 | 97.1 | 87.5 | 88.3 |
| 6 | " | 100 | 94.9 | 84.8 | 87.1 |

CHP/B = Cumene-Hydroperoxide/Borate

Examples 7—13

30 g of the cumene solution containing 20 wt% of cumene-hydroperoxde, which was the same as that used in Example 1; 8.2 g of styrene (twice mols on the basis of said cumene-hydroperoxide); 1/3,000 mol of 10 wt% solution of molybdenum naphthenate; and 1/40 mol of a borate as listed in the following Table 2, were fed in a three neck flat bottom distillation flask having a content volume of 100 ml and provided with a thermometer, a stirrer and a reflux condenser, and were reacted at a reaction temperature of 115°C for a reaction period as determined in Table 2, while heated and stirred.

TABLE 2

| Example No. | Borate | Reaction Time: 10 Minutes: Conversion of CHP (%) | 10 Minutes: Selectivity of Styrene Oxide: To Styrene (%) | 10 Minutes: Selectivity of Styrene Oxide: To CHP (%) | 30 Minutes: Conversion of CHP (%) | 30 Minutes: Selectivity of Styrene Oxide: To Styrene (%) | 30 Minutes: Selectivity of Styrene Oxide: To CHP (%) | 60 Minutes: Conversion of CHP (%) | 60 Minutes: Selectivity of Styrene Oxide: To Styrene (%) | 60 Minutes: Selectivity of Styrene Oxide: To CHP (%) |
|---|---|---|---|---|---|---|---|---|---|---|
| 7 | Triisopropyl Borate | 95.7 | 100 | 92.3 | 98.7 | 100 | 91.5 | 99.5 | 100 | 90.0 |
| 8 | Triisobutyl Borate | 94.6 | 98.0 | 85.0 | 98.3 | 93.8 | 84.7 | 99.1 | 89.0 | 83.7 |
| 9 | Tri-sec-butyl Borate | 94.9 | 100 | 90.5 | 98.5 | 98.0 | 89.8 | 99.3 | 94.0 | 89.3 |
| 10 | Tri-tert-butyl Borate | 95.8 | 100 | 91.8 | 98.6 | 100 | 90.7 | 99.3 | 100 | 90.2 |
| 11 | Tricumyl Borate | 94.7 | 100 | 90.5 | 98.5 | 100 | 90.2 | 99.5 | 100 | 90.1 |
| 12 | Trimethyl Borate | 94.3 | 95.2 | 75.3 | 96.8 | 91.3 | 74.6 | 98.6 | 84.7 | 73.7 |
| 13 | Tri-n-butyl Borate | 94.8 | 94.0 | 78.2 | 97.0 | 88.1 | 76.2 | 98.3 | 85.0 | 75.2 |

CHP = Cumene-Hydroperoxide

Examples 14—16

The same reaction was carried out as in Example 7, with the exception that 10 wtppm of sodium hydroxide was added to the cumene solution containing 20 wt% of cumene-hydroperoxide used in Example 7 (Example 14), that 3 wtppm of sodium hydroxide was added thereto (Example 15), or that a cumene solution containing 20 wt% of cumene-hydroperoxide, prepared from percumyl H (registered trademark, commercial stabilized products containing 81 wt% of cumene-hydroperoxide by Nippon Oils & Fats Co., Ltd.) was used instead of said cumene solution of Example 7 (Example 16). Results are given in the following Table 3.

TABLE 3

| Example No. | Borate | Reaction Time: 10 Minutes: Conversion of CHP (%) | 10 Minutes: Selectivity of Styrene Oxide: To Styrene (%) | 10 Minutes: Selectivity of Styrene Oxide: To CHP (%) | 30 Minutes: Conversion of CHP (%) | 30 Minutes: Selectivity of Styrene Oxide: To Styrene (%) | 30 Minutes: Selectivity of Styrene Oxide: To CHP (%) | 60 Minutes: Conversion of CHP (%) | 60 Minutes: Selectivity of Styrene Oxide: To Styrene (%) | 60 Minutes: Selectivity of Styrene Oxide: To CHP (%) |
|---|---|---|---|---|---|---|---|---|---|---|
| 14 | Triisopropyl Borate | 92.3 | 83.5 | 91.7 | 94.0 | 81.9 | 90.5 | 96.7 | 76.3 | 89.7 |
| 15 | " | 95.2 | 100 | 92.0 | 98.1 | 99.3 | 91.8 | 99.3 | 98.2 | 91.3 |
| 16 | " | 93.5 | 92.1 | 91.8 | 97.8 | 87.8 | 90.7 | 98.6 | 77.6 | 90.1 |

CHP = Cumene-Hydroperoxide

Example 17

0.007 g (0.02 mmol) of molybdenum oxideacetylacetonate, 0.117 g (1.13 mmol) of trimethyl borate, 4.4 g of Percumyl H (registered trademark by Nippon Oils & Fats Co., Ltd.) containing 23.5 mmol of cumene-hydroperoxide, 4.7 g (45.3 mmol) of styrene and 35 ml of ethylbenzene were fed in a three neck flat bottom distillation flask having a content volume of 100 ml and provided with a thermometer, a stirrer and a reflux condenser, and were reacted at 125°C for 30 minutes in nitrogen atmosphere, while heated and stirred.

After the reaction, the reaction solution was cooled, and then, the conversion of the used cumene-hydroperoxide was determined by iodometry, and the amount of each of the non-reacted styrene and reaction products was determined by quantitative analysis with gas chromatography.

As a result, the conversion of the cumene-hydroperoxide was 98.0%, the selectivity of the styrene oxide to the styrene was 100%, and the selectivity thereof to the cumene-hydroperoxide was 74.0%.

Comparative Examples 1—3

The same reaction was carried out as in Example 17, with the exception that an additive listed in the following Table 4 was used instead of the boron compound in Example 17. Results are given in Table 4.

TABLE 4

| Comparative Example No. | Additive | Amount of Additive (g) | Conversion of Cumene-Hydroperoxide (%) | Selectivity of Styrene Oxide: To Styrene (%) | Selectivity of Styrene Oxide: To Cumene-Hydroperoxide (%) |
|---|---|---|---|---|---|
| 1 | None | 0 | 87.7 | 53.7 | 55.1 |
| 2 | Nonylamine | 0.16 | 93.1 | 66.7 | 64.7 |
| 3 | N,N-Dimethylaniline | 0.14 | 90.5 | 68.3 | 65.2 |

Comparative Example 4

The same reaction as in Example 17 was carried out, with the exception that molybdenum oxide-acetyl-acetonate was not used. As a result, the conversion of cumene-hydroperoxide was 10.8%, the selectivity of styrene oxide to styrene was 6.9% and the selectivity thereof to cumene-hydroperoxide was 18.8%.

Examples 18—20

The same reaction as in Example 17 was carried out, with the exception that the reaction time was 10 minutes and that a boron compound as listed in the following Table 5 was used instead of trimethyl borate. Results are given in Table 5.

TABLE 5

| Example No. | Boron Compound | Amount of Boron Compound (g) | Conversion of Cumene-Hydroperoxide (%) | Selectivity of Styrene Oxide To Styrene (%) | Selectivity of Styrene Oxide To Cumene-Hydroperoxide (%) |
|---|---|---|---|---|---|
| 18 | Trimethyl Borate | 0.12 | 96.7 | 100 | 72.0 |
| 19 | Triisopropyl Borate | 0.21 | 97.9 | 100 | 75.1 |
| 20 | Tri-n-butyl Borate | 0.26 | 94.2 | 100 | 76.0 |

Example 21

The same reaction as in Example 17 was carried out, with the exception that the used amount of the ethylbenzene was 14 ml and that the reaction temperature was 115°C.

As a result, the conversion of cumene-hydroperoxide was 95.0%, the selectivity of styrene oxide to styrene was 100%, and the selectivity thereof to cumene-hydroperoxide was 74.3%.

Example 22

The same reaction as in Example 21 was carried out, with the exception that molybdenum naphthenate was used instead of molybdenum oxide-acetylacetonate.

As a result, the conversion of cumene-hydroperoxide was 96.1%, the selectivity of styrene oxide to styrene was 99.3%, and the selectivity thereof to cumene-hydroperoxide was 73.8%.

Example 23

The same reaction as in Example 21 was carried out, with the exception that ethylbenzene-hydroperoxide was used instead of cumene-hydroperoxide.

As a result, the conversion of ethylbenzene-hydroperoxide was 94.7%, the selectivity of styrene oxide to styrene was 100%, and the selectivity thereof to ethylbenzene-hydroperoxide was 74.1%.

Example 24

2.13 mmol/g of styrene, 1.12 mmol/g of 80 wt% of cumene-hydroperoxide (which was a by-product obtained in a process for preparation of acetone and phenol by catalytic oxidation of cumene and which did not contain any stabilizer of an alkaline substance), $1.12 \times 10^{-3}$ mmol/g of molybdenum oxide-acetylacetonate and $6.27 \times 10^{-2}$ mmol/g of trimethyl borate, in the form of a cumene solution, were passed through a glass tube having an inner diameter of 3.2 mm and a content volume of 25 ml, at a reaction temperature of 115°C within a retention time of 10 minutes.

As a result, the conversion of cumene-hydroperoxide was 97.8%, the selectivity of styrene oxide to styrene was 91.5%, and the selectivity thereof to cumene-hydroperoxide was 87.9%.

Example 25

The same reaction as in Example 18 was carried out, with the exception that 0.031 g (0.38 mmol) of sodium acetate was further added, in addition to the boron compound.

As a result, the conversion of cumene-hydroperoxide was 97.3%, the selectivity of styrene oxide to styrene was 100%, and the selectivity thereof to cumene-hydroperoxide was 74.3%.

Example 26

The same reaction as in Example 18 was carried out, with the exception that 0.05 g (0.38 mmol) of nonylamine was further added in addition to the boron compound.

As a result, the conversion of cumene-hydroperoxide was 98.3%, the selectivity of styrene oxide to styrene was 100%, and the selectivity thereof to cumene-hydroperoxide was 75.3%.

Example 27

The same reaction as in Example 18 was carried out, with the exception that 5.5 g (45.3 mmol) of 4-methylstyrene was used instead of styrene.

As a result, the conversion of cumene-hydroperoxide was 98.7%, the selectivity of 4-methylstyrene oxide to 4-methylstyrene was 100%, and the selectivity thereof to cumene-hydroperoxide was 76.3%.

While the invention has been described in detail and with reference to specific embodiments thereof, it will be apparent to one skilled in the art that various changes and modifications can be made therein without departing from the spirit and scope thereof.

## Claims

1. A method for preparation of styrene oxides by reacting a styrene and an organic peroxide in the presence of an epoxidation catalyst and a boron compound, characterized in that said boron compound is selected from borates of formula (I) or metaborates of formula (II) or a mixture thereof

$$B(OR^1)_3 \qquad (I)$$

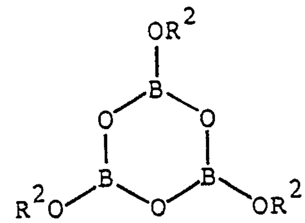

(II)

wherein $R^1$ and $R^2$ are an alkyl group having 1 to 10 carbon atoms, an aralkyl group having 7 to 20 carbon atoms or an aryl group having 6 to 20 carbon atoms.

2. The method for preparation of styrene oxides as claimed in Claim 1, wherein said boron compound is selected from borates of formula (I) or metaborates of formula (II):

$$B(OR^1)_3 \qquad (I)$$

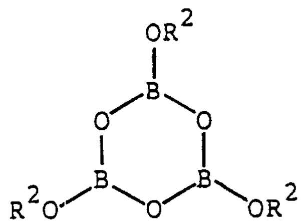

(II)

wherein $R^1$ or $R^2$ is an α-secondary or α-tertiary alkyl group having 3 to 10 carbon atoms or an α-secondary or α-tertiary aralkyl group having 8 to 20 carbon atoms.

3. The method for preparation of styrene oxides as claimed in Claim 1, wherein said boron compound is selected from borates of formula (I):

$$B(OR^1)_3 \qquad (I)$$

wherein $R^1$ is a α-secondary or α-tertiary alkyl group having 3 to 10 carbon atoms or an α-secondary or α-tertiary aralkyl group having 8 to 20 carbon atoms.

4. The method for preparation of styrene oxides as claimed in Claim 1, wherein said epoxidation catalyst is a molybdenum compound.

5. The method for preparation of styrene oxides as claimed in Claim 4, wherein said epoxidation catalyst is a molybdenum compound which is soluble in an organic solvent.

6. The method for preparation of styrene oxides as claimed in Claims 1 to 5, wherein said reaction is carried out in the presence of a hydrocarbon solvent.

7. The method for preparation of styrene oxides as claimed in Claim 1, wherein $R^1$ and $R^2$ each is a branched alkyl group having 3 to 10 carbon atoms, a branched aralkyl group having 8 to 20 carbon atoms or an aryl group having 6 to 20 carbon atoms.

**Patentansprüche**

1. Verfahren zur Herstellung von Styroloxiden durch Umsetzen eines Styrols und eines organischen Peroxids in Gegenwart eines Epoxidationskatalysators und einer Borverbindung, dadurch gekennzeichnet, dass die Borverbindung ausgewählt ist aus Boraten der Formel (I) oder Metaboraten der Formel (II) oder Mischungen daraus

$$B(OR^1)_3 \qquad (I)$$

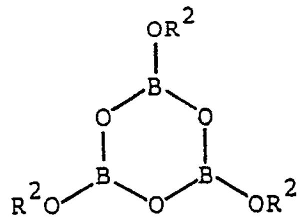

(II)

worin $R^1$ und $R^2$ eine Alkylgruppe mit 1 bis 10 Kohlenstoffatomen, eine Aralkylgruppe mit 7 bis 10 Kohlenstoffatomen oder eine Arylgruppe mit 6 bis 20 Kohlenstoffatomen bedeuten.

2. Verfahren zur Herstellung von Styroloxiden gemäss Anspruch 1, bei dem die Borverbindung von Boraten der Formel (I) oder Metaboraten der Formel (II) ausgewählt ist

$$B(OR^1)_3 \quad (I)$$

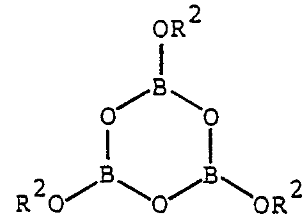

(II)

worin $R^1$ oder $R^2$ eine alpha-sekundäre oder alpha tertiäre Alkylgruppe mit 3 bis 10 Kohlenstoffatomen oder eine alpha-sekundäre oder alpha-tertiäre Aralkylgruppe mit 8 bis 20 Kohlenstoffatomen bedeuten.

3. Verfahren zur Herstellung von Styroloxiden gemäss Anspruch 1, bei dem die Borverbindung aus Boraten der Formel (I) ausgewählt ist

$$B(OR^1)_3 \quad (I)$$

in welcher $R^1$ eine alpha-sekundäre oder alpha-tertiäre Alkylgruppe mit 3 bis 10 Kohlenstoffatomen oder eine alpha-sekundäre oder alpha-tertiäre Aralkylgruppe mit 8 bis 20 Kohlenstoffatomen bedeutet.

4. Verfahren zur Herstellung von Styroloxiden gemäss Anspruch 1, in welchem der Epoxidationskatalysator eine Molbydänverbindung ist.

5. Verfahren zur Herstellung von Styroloxiden gemäss Anspruch 4, bei welchem der Epoxidationskatalysator eine in einem organischen Lösungsmittel lösliche Molybdänverbindung ist.

6. Verfahren zur Herstellung von Styroloxiden gemäss Ansprüchen 1 bis 5, bei welchem man die Umsetzung in Gegenwart eines Kohlenwasserstofflösungsmittels durchführt.

7. Verfahren zur Herstellung von Styroloxiden gemäss Anspruch 1, in welchem $R^1$ und $R^2$ jeweils eine verzweigte Alkylgruppe mit 3 bis 10 Kohlenstoffatomen, eine verzweigte Aralkylgruppe mit 8 bis 20 Kohlenstoffatomen oder eine Arylgruppe mit 6 bis 20 Kohlenstoffatomen bedeuten.

**Revendications**

1. Procédé de préparation d'oxydes de styrène par réaction d'un styrène et d'un peroxyde organique en présence d'un catalyseur d'époxydation et d'un composé de bore, caractérisé en ce que ledit composé de bore est choisi parmi les borates de formule (I) ou les métaborates de formule (II) ou un mélange

$$B(OR^1)_3 \quad (I)$$

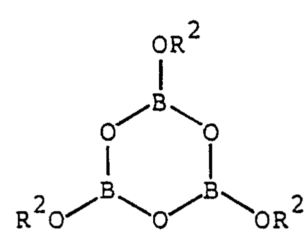

(II)

dans lesquelles $R^1$ et $R^2$ sont un groupe alkyle ayant de 1 à 10 atomes de carbone, un groupe aralkyle ayant de 7 à 20 atoms de carbone ou un groupe aryle ayant de 6 à 20 atomes de carbone.

2. Procédé de préparation d'oxydes de styrène selon la revendication 1, caractérisé en ce que ledit composé de bore est choisi parmi les borates de formule (I) ou les métaborates de formule (II)

$$B(OR^1)_3 \qquad (I)$$

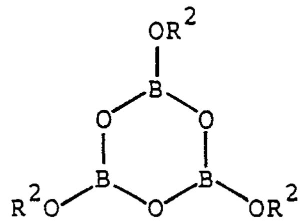

(II)

dans lesquelles $R^1$ ou $R^2$ est un groupe alkyle α-secondaire ou α-tertiaire de 3 à 10 atoms de carbone ou un groupe aralkyle α-secondaire ou α-tertiaire ayant de 8 à 20 atomes de carbone.

3. Procédé de préparation d'oxydes de styrène selon la revendication 1, caractérisé en ce que ledit composé de bore est choisi parmi les borates de formule (I):

$$B(OR^1)_3 \qquad (I)$$

dans laquelle $R^1$ est un groupe alkyle α-secondaire ou α-tertiaire ayant de 3 à 10 atomes de carbone ou un groupe aralkyle α-secondaire ou α-tertiaire ayant de 8 à 20 atomes de carbone.

4. Procédé de préparation d'oxydes de styrène selon la revendication 1, caractérisé en ce que ledit catalyseur d'époxydation est un composé de molybdène.

5. Procédé de préparation d'oxydes de styrène selon la revendication 4, caractérisé en ce que ledit catalyseur d'époxydation est un composé de molybdène qui est soluble dans un solvant organique.

6. Procédé de préparation d'oxydes de styrène selon les revendications 1 à 5, caractérisé en ce que la réaction est réalisée en présence d'un solvant hydrocarboné.

7. Procédé de préparation d'oxydes de styrène selon la revendication 1, caractérisé en ce que $R^1$ et $R^2$ sont chacun un groupe alkyle ramifié ayant de 3 à 10 atomes de carbone, un groupe aralkyle ramifié ayant de 8 à 20 atomes de carbone ou un groupe aryle ayant de 6 à 20 atomes de carbone.